Europäisches Patentamt

European Patent Office

Office européen des brevets

(11) Publication number: **0 140 627 B1**

## EUROPEAN PATENT SPECIFICATION

(45) Date of publication of patent specification: **29.04.92**

(51) Int. Cl.5: **A61K 39/385**, A61K 37/24, A23K 1/165

(21) Application number: **84307007.9**

(22) Date of filing: **12.10.84**

(54) **Production of livestock or products derived therefrom.**

(30) Priority: **14.10.83 GB 8327613**
**14.10.83 GB 8327614**
**14.10.83 GB 8327615**

(43) Date of publication of application:
**08.05.85 Bulletin  85/19**

(45) Publication of the grant of the patent:
**29.04.92 Bulletin  92/18**

(84) Designated Contracting States:
**AT BE CH DE FR GB IT LI NL SE**

(56) References cited:
**EP-A- 0 072 880**
**WO-A-84/03629**

**CHEMICAL ABSTRACTS, vol. 98, no. 3, 17th
January 1983, page 71, abstract no. 11647r,
Columbus, Ohio, US; R.B. LAND et al.:
"Improvement of sheep fecundity by treat-
ment with antiserums to gonadal steroids",
& J. REPROD. FERTIL. 1982, 66(2), 625-34**

(73) Proprietor: **UNILEVER PLC
Unilever House Blackfriars P.O. Box 68
London EC4P 4BO(GB)**

(84) Designated Contracting States:
**GB**

(73) Proprietor: **UNILEVER NV
Burgemeester s'Jacobplein 1 P.O. Box 760
NL-3000 DK Rotterdam(NL)**

(84) Designated Contracting States:
**BE CH DE FR IT LI NL SE AT**

(72) Inventor: **Gill, Richard Davenport
Forge Cottage High Street
Sharnbrook Bedfordshire MK 44 1PF(GB)**
Inventor: **Boyd, John
251 Goldington Road
Bedford(GB)**
Inventor: **Barrat, Michael Edward John
17 Palm Road
Rushden Northamptonshire NN10 9AS(GB)**
Inventor: **Evans, Phillip John
5 Dove Road
Bedford(GB)**

JOURNAL OF DAIRY SCIENCES, vol. 49, no. 4, April 1966, pages 381-385; G.H. SCHMIDT: "Effect of insulin on yield and composition of milk of dairy cows"

(74) Representative: **Butler, David John et al**
**Unilever PLC Patents Division P.O. Box 68**
**Unilever House**
**London EC4P 4BO(GB)**

## Description

The present invention relates to the production of agricultural commodities, especially livestock, and products derived from livestock, such as milk and eggs.

The invention provides a method for enhancing the production of livestock, milk or eggs, in which method an immunogenic hormone conjugate is administered to a female animal to cause the animal to produce systemic antibodies against the hormone, thus altering the hormone balance of the animal and diverting her metabolism to the production of offspring or milk.

In a first aspect, the invention provides a method of rearing animals, in which method the hormone balance of a mother animal is manipulated to divert her metabolism from maternal tissue production to the production of foetal tissue. For the purposes of this specification, the expression "foetal tissue" is taken to mean tissue of or associated with a foetus developing within the mother animal. In its early stages of development a foetus is often referred to as an embryo. Embryonic tissue is thus included within the expression "foetal tissue".

Associated with the developing foetus will be an enveloping mass of tissue, such as the placenta in mammals and the egg contents (yolk and white) in avian species.

In this first aspect, the invention particularly provides a method of rearing animals in which a reproductive female animal is caused to produce antibodies against one or more hormones having an anabolic action on maternal tissue production.

To this end, the invention provides a method of rearing animals in which a reproductive female animal is immunised with a conjugated anabolic hormone. Preferably, this immunisation is effected while the animal is actively producing i.e. in the case of a mammal, during pregnancy, although pre-immunisation can be effected if desired. If the immunisation is effected at or near to the time of conception, there may be a tendency towards abortion or inadequate foetal implantation.

By way of example only, this aspect of the invention will be particularly described in relation to the suppression of insulin production in animals, but it will be appreciated that the suppression of other hormones having a similar anabolic action, such as luteninizing hormone, oestrogen and progesterone, can be accomplished in a similar manner to achieve similar benefits in terms of enhanced foetal production.

Foetus production in a pregnant mammal, for example, can be enhanced by administering to the mother an injectable vaccine comprising conjugated insulin in a pharmaceutically-acceptable diluent.

Examples of mammalian species to which this aspect of the invention can be most suitably applied are ruminants, such as cattle, sheep and goats, and monogastrics such as pigs. The applicability of this aspect of the invention is not limited to mammalian species and, for example, productivity in poultry species such as chickens can be enhanced by the use of an insulin conjugate administered to a laying hen during egg production.

A particular embodiment of the invention is a method of rearing cattle in which a pregnant cow, preferably shortly after conception, is immunised with an insulin conjugate in an amount sufficient to cause the cow to produce systemic antibodies capable of recognising her own insulin.

A further embodiment of the invention is a method of rearing pigs in which a pregnant sow, preferably shortly after conception, is immunised with an insulin conjugate in an amount sufficient to cause the sow to produce systemic antibodies capable of recognising her own insulin.

In a second aspect, the invention particularly provides a method of enhancing milk production, in which a reproductive female mammal is caused to produce antibodies against hormones antagonistic to the beneficial effects of growth hormone.

To this end, the invention provides a method of enhancing milk production in which a female mammal is immunised with a conjugated antagonistic hormone. Preferably, this immunisation should not be effected at or near to the times of conception or parturition.

By way of example only, this second aspect of the invention will be particularly described in relation to the suppression of insulin production in milk-producing animals, but it will be appreciated that the suppression of other antagonistic hormones, such as somatostatin, luteinizing hormone, oestrogen and progesterone, can be accomplished in a similar manner to achieve similar benefits in terms of enhanced milk yield.

Milk production can be enhanced by administering to a mammal an injectable vaccine comprising a conjugate of insulin in a pharmaceutically-acceptable diluent.

Examples of mammalian species to which this second aspect of the invention can be most suitably applied are ruminants, especially cattle, sheep and goats.

3

A particular embodiment of the invention is a method for enhancing milk production, in which method a dairy cow is immunised, preferably after parturition, with an insulin conjugate in an amount sufficient to cause the animal to produce systemic antibodies capable of recognising her own insulin. Suppression of insulin activity in the dairy cow will cause the cow to divert metabolic resources from body tissue development.

The conjugate is required because insulin (like some other hormones) is too small a molecule to possess significant antigenic properties. The conjugate however will be recognised by the mother's immune system and appropriate antibodies will be produced against the conjugate. Some of these antibodies at least will recognise binding sites on the insulin part of the conjugate and will therefore recognise and bind to the same binding sites of the mother's natural insulin circulating within her body, thus rendering the natural insulin inactive.

The hormone can be natural hormone, synthetic hormone made for example by genetic manipulation/cloning techniques, or a synthesised molecule that contains one or more chemical structures that will cause the animal to raise antibodies that react with the natural hormone.

A hormone conjugate can be made by polymerising the hormone itself, e.g. by linking several insulin molecules together by reacting with glutaraldehyde for example. Particulate proteins, such as red blood cells, can be coupled to hormones also by using agents such as glutaraldehyde. Bovine serum albumin is a very good example of a suitable carrier protein with which to conjugate the hormone, but a wide range of proteins can be used. Other examples are ovine serum albumin, ovalbumin and human gamma globulin. The hormone conjugate can be produced by standard techniques, for example those described by Avrameas, S and Ternynck, T in Immunochemistry (1969), vol 6 page 53 et seg.

The diluent in the injectable vaccine can be any pharmaceutically acceptable material, e.g. saline water. Vaccine adjuvants such as Freund's adjuvant, can be included to enhance the animal's response to the vaccine and to provide a delayed release of the immunogenic conjugate. Conjugate-releasing implants can be used.

The degree of hormone imbalance in the body of the mother animal should be such that a beneficial result is obtained in terms of diverting metabolic resources into foetal tissue production or lactation without the health and well-being of the mother animal being significantly affected. The dose level of conjugate administered should be made sufficient to overcome the natural tendency of the animal to step up the production of any hormone of which the activity is being counteracted.

Ideally, administration of the hormone conjugate should be performed not less than about 6 weeks prior to the desired production result, in the case of mammalian species. Thus, for optimum influence on foetal growth, administration should be commenced as soon as possible following conception, preferably within a matter of days. Preferably, administration should not be performed during the implantation period, which (in the sow) occurs about 12-20 days after conception. In the pig, the foetus develops most rapidly after the 70th day of pregnancy.

For optimum milk yield in the dairy cow, administration should be performed not less than about 6 weeks prior to the estimated peak lactation, which is generally about 6-10 weeks after parturition. Administration should therefore commence as soon as possible following parturition, preferably within the first two weeks.

Example 1

The benefit, in terms of enhanced foetal growth and subsequent development, of insulin suppression in pregnant sows is demonstrated by the following experiment. This is contrasted with the suppression of somatostatin, which has a catabolic effect on maternal tissue production. Somatostatin suppression leads to reduced foetal development.

Hormone conjugation

Bovine Serum Albumin (Grade V) (BSA) was used as carrier, with 20 haptenic groups/carrier at 30% coupling efficiency. To give these ratios 28.57 mg BSA/4 mg somatostatin and 34.78 mg BSA/20 mg insulin were required. The BSA, somatostatin and insulin were separately dissolved in 1 ml volumes of phosphate-buffered saline (PBS). The solutions of carrier and hormone are then mixed dropwise with 13 $\mu$l of 25% glutaraldehyde, and incubated at room temperature for 2 hours. A further 1 ml of PBS was then added to the mixture, and then dialysed against PBS at 4°C for up to 48 hours. After dialysis, conjugates were stored at -20°C. Dialysate PBS was assayed by radioimmuno assay (RIA) for the presence of free hormone to estimate coupling efficiency.

4

Treatments

1. Control group - sows were immunized with bovine serum albumin (BSA) and Freund's adjuvant.
2. Anti-somatostatin group - immunized with somatostatin-BSA conjugate and Freund's adjuvant.
3. Anti-insulin group - immunized with insulin-BSA conjugate and Freund's adjuvant.

Immunization Schedule

Conjugate is mixed with an equal volume of Freund's complete adjuvant and emulsified as a water-in-oil suspension. To this emulsion an equal volume of pertussis organisms ($10^{10}$ organisms) was added. The whole mixture was then re-emulsified with an equal volume of 2% "Tween 80". Intra-muscular injection at 4 sites (loins and shoulders) in Freund's complete adjuvant (1st injection only): 600 $\mu$g conjugate per sow (2 ml). 5 administrations at 3-week intervals during gestation. The subsequent administrations were in Freund's incomplete adjuvant, emulsified with "Tween 80".

Trial Organisation

Six sows were randomly allocated into three treatment groups, i.e. anti-insulin, anti-somatostatin, and control. Immunization commenced on Day 9 of gestation, in order to avoid the critical period of implantation (Day 14 to 18), and was repeated at 3-week intervals until Day 93. Sows were blood-sampled (10 ml) on Day 9 and Day 93 of gestation. Animals were housed in a service house until Day 42 of gestation, and then transferred to a dry sow house until farrowing. They were fed a standard commercially-available sow diet at a rate of 2.3 kg per sow per day throughout gestation. During lactation, each sow received 3.5 kg of diet daily. Piglets were weaned at 3 weeks of age. Two piglets from each litter (one male, one female) were sacrificed at birth for carcass analysis.

Results

a) Sow blood analysis

The samples taken on Day 93 revealed that the anti-insulin sows had substantial levels of systemic antibodies specific to insulin, compared to the Control and Day 9 samples. Similarly, on Day 93 the anti-somatostatin sows had substantial levels of systemic antibodies specific to somatostatin.

b) Reproductive performance (Table 1)

Litter size:

There were no significant differences between treatments in the total number of piglets born or the number born alive. Piglets from anti-insulin sows were heavier at birth and at weaning in comparison to the other two treatments.

c) Growth Performance

Weaner period:

Daily liveweight gain was greater in the anti-insulin piglets from 3 to 5 weeks of age, compared to the control and anti-somatostatin treatments.

Grower period:

Growth performance followed a similar pattern in the grower period from 5 to 10 weeks of age, with the anti-insulin pigs growing faster and the anti-somatostatin pigs growing slower in comparison to the control group.

d) Piglet carcass composition:

There were no differences in organ weights or carcass composition between any of the treatment groups. (See Tables 2 and 3 below).

Table 1

| Reproductive performance data | | | |
|---|---|---|---|
| | Control | Anti-Somatostatin | Anti-Insulin |
| Total number born (per litter) | 11.5 | 11.5 | 10.0 |
| Number born alive (per litter) | 11.5 | 9.5 | 10.0 |
| Average birth weight (kg) | 0.98 | 1.02 | 1.39 |
| Total litter weight (kg) | 14.7 | 11.8 | 13.85 |
| Average weaned weight (21 d) | 5.75 | 4.17 | 6.21 |
| Total gain (weaned weight -birth weight) | 4.27 | 3.15 | 4.82 |
| Daily liveweight gain in weaner period (3-5 weeks, gram per day) | 188.4 | 128.3 | 281.9 |
| Daily liveweight gain in grower period (5-10 weeks, gram per day) | 524.4 | 485.5 | 648.5 |

Table 2

| Relative organ weights of piglets expressed as gram/kilogram body weight on a wet tissue basis | | | |
|---|---|---|---|
| TREATMENT | CONTROL | ANTI-SOMATOSTATIN | ANTI-INSULIN |
| Liver | 28.7 | 27.9 | 30.0 |
| Heart | 6.84 | 6.15 | 6.58 |
| Lateral dorsi | 12.9 | 11.4 | 11.8 |

Table 3

| Piglet carcass analysis (% by weight) | | | |
|---|---|---|---|
| | CONTROL | ANTI-SOMATOSTATIN | ANTI-INSULIN |
| Moisture | 82.2 | 81.8 | 82.3 |
| Oil | 1.4 | 1.4 | 1.6 |
| Protein | 11.1 | 11.3 | 11.4 |
| Ash | 3.8 | 4.0 | 3.6 |

Example 2

The benefit, in terms of enhanced milk yield, of insulin suppression in the lactating dairy cow is demonstrated by the following experiment.

Hormone conjugation

Ovalbumin is used as a carrier, with 20 haptenic groups/carrier at 30% coupling efficency. To give this ratio 34.78 mg OA/20 mg insulin are required. The OA and insulin are separately dissolved in 1 ml of PBS. The solutions of carrier and hormone are mixed dropwise with 13 $\mu$l of 25% glutaraldehyde, and incubated at room temperature for 2 hours. A further 1 ml of PBS is added to the mixture, and then dialysed against PBS at 4°C for up to 48 hours. After dialysis, conjugates can be stored at -20°C. Dialysate PBS can be assayed by RIA for the presence of free hormone to estimate coupling efficiency.

6

Treatments

    1. Control group - dairy cows are immunized with ovine serum albumin (OA) and Freund's adjuvant.
    2. Anti-insulin group - immunized with insulin-OA conjugate and Freund's adjuvant.

Immunization Schedule

Conjugate is mixed with an equal volume of Freund's complete adjuvant, and emulsified as a water-in-oil suspension. To this emulsion an equal volume of pertussis organisms ($10^{10}$ organisms) is added. The whole mixture is re-emulsified with an equal volume of 2% "Tween 80". Intra-muscular injection at 4 sites (loins and shoulders) in Freund's complete adjuvant (1st injection only): 1 mg conjugate per cow (2 ml). 3 administrations at 3-week intervals during lactation. The subsequent administrations are in Freund's incomplete adjuvant, emulsified with "Tween 80".

Trial Organisation

A number of dairy cows are randomly allocated into two treatment groups, i.e. anti-insulin and control. Immunization commences and is repeated at 3-week intervals until week 6. Cows are blood-sampled in weeks 1 and 9. The Cows are fed a standard commercially-available diet at a recommended rate throughout lactation.

Results

The blood samples taken on week 9 will reveal that the anti-insulin cows have substantial levels of systemic antibodies specific to insulin, compared to the Control and week 1 samples.

The post peak lactation milk yield from the anti-insulin group of cows will be greater than that from the control group.

**Claims**

1.  A method for enhancing the production of livestock, milk or eggs, in which method an immunogenic hormone conjugate is administered to a non human female animal to cause the animal to produce systemic antibodies against the hormone, thus altering the hormone balance of the animal and diverting her metabolism to the production of offspring or milk.

2.  A method of rearing animals, in which method the hormone balance of a reproductive non human female animal is manipulated to divert her metabolism from maternal tissue production to the production of foetal tissue, by causing the animal to produce antibodies against one or more hormones having an anabolic action on maternal tissue production.

3.  A method according to claim 2, in which the non human animal is immunised with a conjugated anabolic hormone.

4.  A method according to claim 3, in which the non human animal is a mammal.

5.  A method according to any one of claims 2 to 4, in which the anabolic hormone is insulin.

6.  A method of rearing cattle, in which a pregnant cow is immunised with an insulin conjugate in an amount sufficient to cause the cow to produce systemic antibodies capable of recognising her own insulin.

7.  A method of rearing pigs, in which a pregnant sow is immunised with an insulin conjugate in an amount sufficient to cause the sow to produce systemic antibodies capable of recognising her own insulin.

8.  A method of enhancing egg production in poultry, in which a laying hen is immunised with an insulin conjugate in an amount sufficient to cause the hen to produce systemic antibodies capable of recognising her own insulin.

9. A method of enhancing milk production, in which a reproductive non human female mammal is caused to produce antibodies against hormones antagonistic to the beneficial effects of growth hormone.

10. A method according to claim 9, in which the non human mammal is immunised with a conjugated antagonistic hormone.

11. A method according to claim 9 or claim 10, in which the antagonistic hormone is insulin.

12. A method for enhancing milk production, in which method a diary cow is immunised, preferably within two weeks following parturition, with an insulin conjugate in an amount sufficient to cause the cow to produce systemic antibodies capable of recognising her own insulin.

13. Use of a hormone conjugate in the form of an injectable or implantable composition, in a method according to any one of the preceding claims.

14. Use of an insulin conjugate in the form of an injectable or implantable composition, in a method according to any one of claims 1 to 12.

**Revendications**

1. Un procédé pour augmenter la production de bétail, de lait ou d'oeufs, procédé dans lequel on administre un conjugué d'hormone immunogène à un animal femelle non humain afin que l'animal produise des anticorps systémiques contre l'hormone, ce qui altère l'équilibre hormonal de l'animal et oriente son métabolisme vers la production de descendants ou de lait.

2. Un procédé pour élever des animaux, procédé dans lequel l'équilibre hormonal d'un animal reproducteur femelle non humain est manipulé afin d'orienter son métabolisme de la production de tissus maternels vers la production de tissus foetaux, en faisant produire à l'animal des anticorps contre une ou plusieurs hormones ayant une action anabolisante sur la production de tissu maternel.

3. Un procédé selon la revendication 2, dans lequel l'animal non humain est immunisé avec un conjugué d'hormones anabolisantes.

4. Un procédé selon la revendication 3, dans lequel l'animal non humain est un mammifère.

5. Un procédé selon l'une des revendications 2 à 4, dans lequel l'hormone anabolisante est de l'insuline.

6. Un procédé pour élever des bovins, dans lequel on immunise une vache en gestation avec un conjugué d'insuline dans une quantité suffisante pour que cela fasse produire à la vache des anticorps systémiques capables de reconnaître sa propre insuline.

7. Un procédé pour élever des porcins, dans lequel on immunise une truie en gestation avec un conjugué d'insuline dans une quantité suffisante pour que cela fasse produire à la truie des anticorps systémiques capables de reconnaître sa propre insuline.

8. Un procédé pour augmenter la production d'oeufs chez les volailles, dans lequel on immunise une poule pondeuse avec un conjugué d'insuline dans une quantité suffisante pour que cela fasse produire à la poule des anticorps systémiques capables de reconnaître sa propre insuline.

9. Un procédé pour augmenter la production laitière, dans lequel on fait produire à un animal femelle reproducteur non humain des anticorps contre les hormones antagonistes par rapport aux effets bénéfiques de l'hormone de croissance.

10. Un procédé selon la revendication 9, dans lequel le mammifère non humain est immunisé avec un conjugué d'hormone antagoniste.

11. Un procédé selon la revendication 9 ou la revendication 10, dans lequel l'hormone antagoniste est de l'insuline.

**12.** Un procédé pour augmenter la production laitière, procédé dans lequel on immunise une vache laitière, de préférence dans les deux semaines après la mise bas, avec un conjugué d'insuline dans une quantité suffisante pour que cela fasse produire à la vache des anticorps systémiques capables de reconnaître sa propre insuline.

**13.** Utilisation d'un conjugué d'hormone sous forme d'une composition injectable ou implantable, selon un procédé en accord avec l'une des revendications précédentes.

**14.** L'utilisation d'un conjugué d'insuline sous forme d'une composition injectable ou implantable, selon un procédé en accord avec l'une des revendications 1 à 12.

**Patentansprüche**

**1.** Verfahren zum Erhöhen der Produktion von Vieh, Milch oder Eiern, in welchem Verfahren ein immunogenes Hormonkonjugat einem nicht-menschlichen weiblichen Tier verabreicht wird, um zu bewirken, daß das Tier systemische Antikörper gegen das Hormon produziert, wodurch das Hormongleichgewicht des Tieres verändert und sein Metabolismus auf die Produktion von Nachkommen oder Milch gelenkt wird.

**2.** Verfahren zum Züchten von Tieren, in welchem Verfahren das Hormongleichgewicht eines fortpflanzungsfähigen nicht-menschlichen weiblichen Tieres manipuliert wird, um seinen Metabolismus von der Muttergewebeproduktion auf die Produktion von Fötusgewebe zu lenken, indem bewirkt wird, daß das Tier Antikörper gegen ein oder mehrere Hormone mit einer anabolischen Wirkung auf die Muttergewebeproduktion produziert.

**3.** Verfahren nach Anspruch 2, indem das nicht-menschliche Tier mit einem konjugierten anabolischen Hormon immunisiert wird.

**4.** Verfahren nach Anspruch 3, in dem das nicht-menschliche Tier ein Säugetier ist.

**5.** Verfahren nach einem der Ansprüche 2 bis 4, in dem das anabolische Hormon Insulin ist.

**6.** Verfahren zum Züchten von Rindern, in dem eine trächtige Kuh mit einem Insulinkonjugat in einer Menge immunisiert wird, die ausreichend ist zu bewirken, daß die Kuh systemische Antikörper produziert, die imstande sind, ihr eigenes Insulin zu erkennen.

**7.** Verfahren zum Züchten von Schweinen, in dem eine trächtige Sau mit einem Insulinkonjugat in einer Menge immunisiert wird, die ausreichend ist zu bewirken, daß die Sau systemische Antikörper produziert, die imstande sind, ihr eigenes Insulin zu erkennen.

**8.** Verfahren zum Erhöhen der Eiproduktion in Geflügel, in dem eine Legehenne mit einem Insulinkonjugat in einer Menge immunisiert wird, die ausreichend ist zu bewirken, daß die Henne systemische Antikörper produziert, die imstande sind, ihr eigenes Insulin zu erkennen.

**9.** Verfahren zum Erhöhen der Milchproduktion, in dem ein fortpflanzungsfähiges nicht-menschliches weibliches Säugetier veranlaßt wird, Antikörper gegen Hormone zu produzieren, die für die günstigen Wirkungen von Wachstumshormon antagonistisch sind.

**10.** Verfahren nach Anspruch 9, in dem das nicht-menschliche Säugetier mit einem konjugierten antagonistischen Hormon immunisiert wird.

**11.** Verfahren nach Anspruch 9 oder 10, in dem das antagonistische Hormon Insulin ist.

**12.** Verfahren zum Erhöhen der Milchproduktion, in welchem Verfahren eine Milchkuh, vorzugsweise innerhalb von zwei Wochen nach der Niederkunft, mit einem Insulinkonjugat in einer Menge immunisiert wird, die ausreichend ist zu bewirken, daß die Kuh systemische Antikörper produziert, die imstande sind, ihr eigenes Insulin zu erkennen.

**13.** Verwendung eines Hormonkonjugatz in Form einer injizierbaren oder implantierbaren Zusammensetzung in einem Verfahren nach einem der vorhergehenden Ansprüche.

**14.** Verwendung eines Insulinkonjugats in Form einer injizierbaren oder implantierbaren Zusammensetzung in einem Verfahren nach einem der Ansprüche 1 bis 12.